# EUROPÄISCHE PATENTSCHRIFT

(11) **EP 1 202 704 B1**
(45) Veröffentlichungstag und Bekanntmachung des Hinweises auf die Patenterteilung: **13.07.2005**
(21) Anmeldenummer: 00949431.1
(22) Anmeldetag: 01.08.2000
(51) Int. Cl.: A61K 7/13

(54) **VERFAHREN ZUR FÄRBUNG KERATINISCHER FASERN**
METHOD FOR DYEING FIBRES CONTAINING KERATIN
PROCEDE POUR COLORER DES FIBRES KERATINIQUES

(30) Priorität: 10.08.1999 DE 19937311
(43) Veröffentlichungstag der Anmeldung: 08.05.2002
(73) Patentinhaber: Henkel Kommanditgesellschaft auf Aktien, 40589 Düsseldorf (DE)
(72) Erfinder: OBERKOBUSCH, Doris, D-40591 Düsseldorf (DE); HOLLENBERG, Detlef, D-40699 Erkrath (DE); HÖFFKES, Horst, D-40595 Düsseldorf (DE); ERKENS, Udo, D-41468 Neuss (DE); KLEEN, Astrid, D-40699 Erkrath (DE)
(86) Internationale Anmeldenummer: PCT/EP2000/007428
(87) Internationale Veröffentlichungsnummer: WO 2001/010399

(56) Entgegenhaltungen:
- EP-A- 0 661 040
- WO-A-97/24107
- DE-A- 2 819 036
- US-A- 5 441 542

## Beschreibung

Die Erfindung betrifft ein Verfahren zum Färben keratinischer Fasern.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung:

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Weisen die im Verlauf der Farbausbildung gebildeten bzw. direkt eingesetzten Farbstoffe deutlich unterschiedliche Echtheiten (z. B. UV-Stabilität, Schweißechtheit. Waschechtheit etc.) auf, so kann es mit der Zeit zu einer erkennbaren und daher unerwünschten Farbverschiebung kommen. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so daß dann sehr viel schneller eine vielfach unerwünschte Nuancenverschiebung oder gar eine sichtbare "Entfärbung" eintritt.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher. Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden. Auch hier können dann Probleme hinsichtlich der Echtheit der Färbungen auftreten.

Es hat daher nicht an Anstrengungen gefehlt, die Echtheit von Färbungen keratinischer Fasern zu verbessern. Eine Entwicklungsnchtung ist die Optimierung der Farbstoffe selbst bzw. die Synthese neuer, modifizierter Farbstoffmoleküle. Eine weitere Entwicklungsrichtung ist die Suche nach Zusätzen für die Färbemittel, um die Echtheit der Färbungen zu erhöhen. Eine bekannte Problemlösung ist, dem Färbemittel UV-Filter zuzusetzen. Diese Filtersubstanzen werden beim Färbeprozeß zusammen mit dem Farbstoff auf das Haar aufgebracht, wodurch in vielen Fällen eine deutliche Steigerung der Stabilität der Färbung gegen die Einwirkung von Tages- oder Kunstlicht erzielt wird. Außerdem erhöhen die UV-Filter, wie in der DE-A1-198 53 111 beschrieben, auch die Waschechtheit der Färbungen.

Es wurde nun überraschenderweise gefunden, daß die Waschechtheit von Färbungen keratinischer Fasern deutlich erhöht werden kann, in dem man die Faser im Anschluß an den Färbeprozeß mit einer Zubereitung eines Oxidationsmittels behandelt. Unter Waschechtheit im Sinne der Erfindung ist die Erhaltung der ursprünglichen Färbung hinsichtlich Nuance und/oder Intensität zu verstehen, wenn die keratinische Faser dem wiederholten Einfluß von wäßrigen Mitteln, insbesondere tensidhaltigen Mitteln wie Shampoos, ausgesetzt wird.

Ein erster Gegenstand der vorliegenden Erfindung ist daher ein Verfahren zur Färbung keratinischer Fasern gemäß Anspruch 1, dadurch gekennzeichnet, daß in einem ersten Schritt die keratinischen Fasern in üblicher Weise gefärbt werden (eigentlicher Färbevorgang) und in einem zweiten Schritt eine Zubereitung auf die keratinischen Fasern aufgetragen wird, die ein Oxidationsmittel enthält, wobei die Zubereitung unmittelbar nach der Anwendung oder nach einer kurzen Einwirkzeit wieder ausgespült wird.

Unter keratinischen Fasern werden erfindungsgemäß Pelze, Wolle, Federn und insbesondere menschliche Haare verstanden.

Das im Rahmen des zweiten Schrittes des erfindungsgemäßen Verfahrens auf die Faser aufgebrachte Oxidationsmittel unterliegt keinen prinzipiellen Beschränkungen. Bei der Färbung menschlicher Haare wird es selbstverständlich in Art und Konzentration so gewählt werden, daß es physiologisch verträglich ist, also keine Schäden oder Irritationen auf Haar und Kopfhaut verursacht.

Bevorzugt weist das Oxidationsmittel bei einer Aktivität von 1, dem pH-Wert von 7 und einer Temperatur von 25 °C ein Redoxpotential von mindestens + 0,4 V gegenüber der Normalwasserstoffelektrode auf.

Gemäß einer ersten bevorzugten Ausführungsform des erfindungsgemäßen Verfahrens ist das Oxidationsmittel Wasserstoffperoxid.

Gemäß einer zweiten Ausführungsform handelt es sich bei dem Oxidationsmittel um eine organische Verbindung, Bevorzugte organische Verbindungen sind
- Benzochinone, wie z. B. 1,4-Benzochinon. 2-Methyl-1,4-benzochinon, 2,6-Dimethyl-1,4-benzochinon, 2-Chlor-1,4-benzochinon. 2-Dimethylamino-1,4-benzochinon, 2,3-Dimethyl-1,4-benzochinon, 1,4-Naphthochinon und 1,2-Naphthochinon,
- Percarbamid,
- Anlagerungsprodukte von Wasserstoffperoxid an Melamin. Polyvinylpyrrolidon und Harnstoff,
- Flavinderivate und
- Azodicarbonamid.

Besonders bevorzugte organische Verbindungen sind Benzochinone, Percarbamid und Melaminperhydrat.

Gemäß einer dritten Ausführungsform kann das Oxidationsmittel auch in der Zubereitung in situ mit Hilfe von Enzymen gebildet werden. Dabei sind prinzipiell zwei Vorgehensweisen möglich. Zum einen wird das Oxidationsmittel direkt in situ durch die Umsetzung eines Enzyms mit seinem Substat erzeugt. Beispiele für entsprechende Enzyme sind Oxidasen, wie Glucoseoxidase, Laccasen, Uricase und Tyrosinase. Zum anderen kann die Wirkung einer geringen Menge eines bereits vorliegenden oder von einem Enzym gebildeten Oxidationsmittels durch ein weiteres Enzym. z. B. eine Peroxidase, verstärkt werden.

Die Menge des Oxidationsmittels in der Zubereitung beträgt 0,1 - 10 Gew.-%, bezogen auf die gesamte Zubereitung. Mengen von 0,5 - 5 % haben sich als besonders geeignet erwiesen. Für den Fall, daß das Oxidationsmittel unter Mitwirkung von Enzymen gebildet wird, wird die eingesetzte Menge im unteren Teil des genannten Bereiches liegen (wenn die Wirkung durch das Enzym verstärkt wird) bzw. kann während der Einwirkungszeit auch geringfügig unterhalb von 0.1 Gew.-% liegen.

Die Art der Zubereitung des Oxidationsmittels unterliegt keinen prinzipiellen Einschränkungen. Erfindungsgemäß geeignet sind insbesondere wäßrige, alkoholische und ölige Zubereitungen sowie deren Mischungen. Besonders bevorzugt sind wäßrige Zubereitungen. Es kann sich beispielsweise um Lösungen, Dispersionen, Emulsionen (Wasser in Öl-Emulsionen, Öl in Wasser-Emulsionen sowie multiple Emulsionen und PIT-Emulsionen) handeln. Der pH-Wert dieser Zubereitungen liegt in der Regel bei 2 bis 9. bevorzugt bei 3 bis 7 und besonders bevorzugt bei 4 bis 6.

Diese Zubereitungen werden üblicherweise nach der eigentlichen Haarfarbung, d. h. nach dem Ausspülen des Oxidationsfarbemittel, dem Ausspülen des Färbemittels mit naturanalogen Farbstoffvorprodukten oder dem Aufbringen des nur direktziehende Farbstoffe enthaltenden Tönungsmittels, auf das noch feuchte Haar aufgebracht. Obwohl die Zubereitung prinzipiell unmittelbar nach ihrer Anwendung wieder ausgespült werden kann, wird in einer bevorzugten Ausführungsform des erfindungsgemäßen Verfahrens die Zubereitung, die das Oxidationsmittel enthält, nach einer Einwirkzeit von 1 bis 15 Minuten ausgespült. Dieses Ausspülen kann mit reinem Wasser erfolgen. Einwirkzeiten von 2 bis 5 Minuten haben sich in den meisten Fällen als ausreichend erwiesen.

Die farberhaltende Wirkung der Zubereitung des Oxidationsmittels wird aber auch beobachtet, wenn diese zu einem späteren Zeitpunkt nach dem Färben. z. B. vor oder nach den üblichen Haarwäschen, angewendet wird.

Die Zubereitungen, die im weiteren als "Farbfixiermittel" bezeichnet werden, können außer Oxidationsmittel, ggf. Enzym und Substrat, alle üblichen Bestandteile enthalten, die für die Behandlung keratinischer Fasern, insbesondere menschlicher Haare, geeignet sind. Bevorzugt sind wäßrige Zubereitungen. Unter wäßrigen Zubereitungen werden im Rahmen der Erfindungen solche Mittel verstanden, die mindestens 50 Gew.-% Wasser, bezogen auf das gesamte Mittel, enthalten.

Es hat sich als vorteilhaft erwiesen, wenn die Farbfixiermittel mindestens ein Tensid enthalten. Es kann sich dabei sowohl um anionische, ampholytische, zwitterionische oder mchtionogene Tenside als auch um kationische Tenside handeln.

Als anionische Tenside eignen sich alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium-, Magnesium- und Ammomum- sowie der Mono-, D₁- und Trialkanolammoniumsalze mit 2 oder 3 C-Atomen in der Alkanolgruppe.
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Amidethercarboxylate der Formel [R-NH(-CH₂-CH₂-O)ₙ-CH₂-COO]ₘZ, in der R für einen linearen oder verzweigten, gesättigten oder ungesättigten Acylrest mit 2 bis 29 C-Atomen, n für ganze Zahlen von 1 bis 10, m für die Zahlen 1 oder 2 und Z für ein Kation aus der Gruppe der Alkali- oder Erdalkalimetalle steht,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Kokosmonoglyceridsulfate.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen sowie Seifen.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel RO-(Z)ₓ. Diese Verbindungen sind durch die folgenden Parameter gekennzeichnet.

Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt smd 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur emen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R
- im wesentlichen aus C₈- und C₁₀-Alkylgruppen,
- im wesentlichen aus C₁₂- und C ₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, daß eine über die Dauer der Haarbehandlung hinausgehende Wirkung des Parfümöles auf dem Haar gewünscht wird, bevorzugt zu dieser Substanzklasse als weiterem Inhaltsstoff zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Ammogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen. Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide. -hydroxide oder -alkoholate als Katalysatoren verwendet werden. Die Verwendung von Produkten mit eingeengter Homologenverteilung kann bevorzugt sein.

Weiterhin enthalten die erfindungsgemäß verwendeten Farbfixiermittel bevorzugt mindestens eine Ölkomponente.

Erfindungsgemäß geeignete Ölkomponenten sind prinzipiell alle wasserunlöslichen Öle und Fettstoffe sowie deren Mischungen mit festen Paraffinen und Wachsen. Als wasserunlöslich werden erfindungsgemäß solche Stoffe definiert, deren Löslichkeit in Wasser bei 20 °C kleiner als 0.1 Gew,-% betragt. Der Schmelzpunkt der einzelnen Öl- oder Fettkomponenten liegt bevorzugt unterhalb von etwa 40 °C. Öl- und Fettkomponenten, die bei Raumtemperatur, d. h. unterhalb von 25 °C flüssig sind, können erfindungsgemäß besonders bevorzugt sein. Bei Verwendung mehrerer Öl- und Fettkomponenten sowie ggf. festen Paraffinen und Wachsen ist es in der Regel jedoch auch ausreichend, wenn die Mischung der Öl- und Fettkomponenten sowie ggf. Paraffine und Wachse diesen Bedingungen genügt.

Eine bevorzugte Gruppe von Ölkomponenten sind pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls.

Geeignet sind aber auch andere Triglyceridöle wie die flüssigen Anteile des Rindertalgs sowie synthetische Triglyceridöle.

Eine weitere, besonders bevorzugte Gruppe erfindungsgemäß als Ölkomponente einsetzbarer Verbindungen sind flüssige Paraffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether. Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1.3-Di-(2-ethyl-hexyl)-cyclohexan (Cetiol® S) und Di-n-octylether (Cetiol® OE) können bevorzugt sein.

Ebenfalls erfindungsgemäß einsetzbare Ölkomponenten sind Fettsäure- und Fettalkoholester. Bevorzugt sind die Monoester der Fettsäuren mit Alkoholen mit 3 bis 24 C-Atomen. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen, bei der Reduktion von Aldehyden aus der Roelen'schen Oxosynthese oder der Dimerisierung von ungesättigten Fettsäuren anfallen, mit Alkoholen wie beispielsweise Isopropylalkohol, Capronalkohol. Caprylalkohol. 2-Ethylhexylalkohol, Caprinalkohol, Laurylalkohol, Isotndecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydnerung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat, Isononansäure-C16-18-alkylester (Cetiol® SN), Stearinsäure-2-ethylhexylester (Cetiol® 868), Cetyloleat. Glycerintricaprylat, Kokosfettalkohol-caprinat/-caprylat und n-Butylstearat.

Weiterhin stellen auch Dicarbonsäureester wie Di-n-butyladipat, Di-(2-ethylhexyl)-adipat, Di-(2-ethylhexyl)-succinat und Di-isotridecylacelaat sowie Diolester wie Ethylenglykoldioleat, Ethylenglykol-di-isotridecanoat, Propylenglykol-di(2-ethylhexanoat), Propylenglykol-di-isostearat, Propylenglykol-di-pelargonat, Butandiol-di-isostearat und Neopentylglykoldi-capylat erfindungsgemäß verwendbare Ölkomponenten dar, ebenso komplexe Ester wie z. B. das Diacetyl- glycennmonostearat.

Schließlich können auch Fettalkohole mit 8 bis 22 C-Atomen als erfindungsgemäß wirkende Ölkomponenten eingesetzt werden. Die Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohoi und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen.

Die erfindungsgemäß verwendeten Farbfixiermittel enthalten gemäß einer bevorzugten Ausführungsform einen Pflegestoff. Dieser Pflegestoff ist bevorzugt ausgewählt aus der Gruppe, die von kationischen Polymeren, Silikonen und Proteinhydrolysaten sowie deren Derivaten gebildet wird.

Eine erste Gruppe von kationischen Polymeren sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Unter den kationischen Polymeren sind aber die permanent kationischen Polymere bevorzugt. Als "permanent kationisch" werden erfindungsgemäß solche Polymeren bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80),
- Kationische Guar-Denvate, wie insbesondere die unter den Handelsnamen Cosmedia®Guar und Jaguar® vertriebenen Produkte,
- Polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden.
- quaternierter Polyvinylalkohol,
- sowie die unter den Bezeichnungen
- Polyquaternium 2,
- Polyquatemium 17,
- Polyquatemium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), Polyquaternium-32, Polyquaternium-35 und Polyquaternium-37 (Handelsprodukte z. B. Salcare® SC 92 und Saicare®SC 95) bekannten Polymere. Ebenfalls erfindungsgemäß verwendbar sind die Copolymere des Vinylpyrrolidons, wie sie als Handelsprodukte Copolymer 845 (Hersteller: ISP). Gaffix® VC 713 (Hersteller: ISP), Gafquat®ASCP 1011, Gafquat®HS 110. Luviquat®8155 und Luviquat® MS 370 erhältlich sind.

Erfindungsgemäß bevorzugte kationische Polymere sind quaternisierte Cellulose-Derivate, polymere Dimethyldiallylammoniumsalze, Polyquaternium-27 und deren Copolymere sowie Polymere vom Typ Polyquaterniuim-2. Kationische Cellulose-Derivate, insbesondere das Handelsprodukt Polymer®JR 400, und Polymere vom Typ Polyquaternium-2. insbesondere das Handelsprodukt Mirapol®A-15, sind ganz besonders bevorzugte kationische Polymere.

Die kationischen Polymeren sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Geeignet als Pflegestoff in Kombination mit oder alternativ zu kationischen Polymeren sind auch Ampho-Polymere. Unter dem Oberbegriff Ampho-Polymere sind amphotere Polymere. d. h. Polymere, die im Molekül sowohl freie Aminogruppen als auch freie - COOH- oder SO₃H-Gruppen enthalten und zur Ausbildung innerer Salze befähigt sind, zwitterionische Polymere, die im Molekül quartäre Ammoniumgruppen und -COO⁻- oder -SO₃⁻-Gruppen enthalten, und solche Polymeren zusammengefaßt, die -COOH- oder SO₃H-Gruppen und quartäre Ammoniumgruppen enthalten. Ein Beispiel für ein erfindungsgemäß einsetzbares Amphopolymer ist das unter der Bezeichnung Amphomer® erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt. Ebenfalls bevorzugte Amphopolymere setzen sich aus ungesättigten Carbonsäuren (z. B. Acryl- und Methacrylsäure), kationisch derivatisierten ungesättigten Carbonsäuren (z. B. Acrylamidopropyl-trimethyl-ammoniumchlorid) und gegebenenfalls weiteren ionischen oder nichtionogenen Monomeren zusammen, wie beispielsweise in der deutschen Offenlegungsschrift 39 29 973 und dem dort zitierten Stand der Technik zu entnehmen sind. Terpolymere von Acrylsäure, Methylacrylat und Methacrylamidopropyltrimoniumchlorid, wie sie unter der Bezeichnung Merquat®2001 N im Handel erhältlich sind, sind erfindungsgemäß besonders bevorzugte Ampho-Polymere.

Erfindungsgemäß verwendbare Pflegestoffe sind weiterhin Silikonöle und Silikon-Gums, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Coming unter den Bezeichnungen DC 190, DC 200 und DC 1401 vertriebenen Produkte sowie die Handelsprodukte DC 344 und DC 345 von Dow Coming, Q2-7224 (Hersteller: Dow Coming: ein stabilisiertes Trimethylsilylamodimethicon), Dow Corning® 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80) und das Handelsprodukt Fancorsil® LIM-1. Ein geeignetes anionisches Silikonöl ist das Produkt Dow Corning®1784.

Poteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Henkel), Promois® (Interorgana), Collapuron® (Henkel), Nutrilan® (Grünau), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Erbsen-, Kartoffel- und Weizenproteinhydrolysate. Solche Produkte sind beispielsweise unter den Warenzeichen Gluadin® (Henkel), DiaMin® (Diamalt), Lexein® (Inolex) und Crotein® (Croda) erhältlich.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich, wenngleich wemger bevorzugt, ist der Einsatz von Derivaten der Proteinhydrolysate, beispielsweise in Form ihrer Fettsäure-Kondensationsprodukte oder kationisch denvatisiert. Solche Produkte werden beispielsweise unter den Bezeichnungen Lamepon® (Henkel), Lexein® (Inolex), Crolastin® (Croda), Crotein® (Croda), Lamequat® und Croquat® vertrieben.

Die Proteinhydrolysate oder deren Derivate sind in den erfindungsgemäß verwendeten Farbfixiermitteln bevorzugt in Mengen von 0,1 bis 5 Gew.-%, bezogen auf das gesamte Mittel, enthalten. Mengen von 0,1 bis 2 Gew.-% sind besonders bevorzugt.

Neben dem Oxidationsmittel und den weiteren, oben genannten bevorzugten Komponenten können die Farbfixiermittel prinzipiell alle weiteren, dem Fachmann für solche kosmetischen Mittel bekannten Komponenten enthalten.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- Verdickungsmittel wie Agar-Agar, Guar-Gum, Alginate, Xanthan-Gum, Gummi arabicum, Karaya-Gummi, Johannisbrotkernmehl. Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Maleinsäure und Milchsäure,
- haarkonditionierende Verbindungen wie Phospholipide, beispielsweise Sojalecithin. Ei-Lecitin und Kephaline,
- Parfümöle, Dimethylisosorbid und Cyclodextrine,
- Lösungsmittel und -Vermittler wie Ethanol, Isopropanol. Ethylenglykol, Propylenglykol, Glycerin und Diethylenglykol.
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels.
- Antischuppenwirkstoffe wie Piroctone Olamine. Zink Omadine und Climbazol.
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, C, E, F und H,
- Pflanzenextrakte wie die Extrakte aus Grünem Tee, Eichenrinde, Brennessel. Hamamelis, Hopfen, Kamille, Klettenwurzel. Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit. Salbei, Rosmarin, Birke, Malve, Wiesenschaumkraut, Quendel, Schafgarbe. Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren.
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO, und Luft..

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Das Aufbringen des Farbfixiermittels auf die gefärbte keratinische Faser erfolgt in einem getrennten Schritt entweder direkt im Anschluß an den eigentlichen Färbevorgang oder in getrennten Behandlungen, gegebenenfalls auch Tage oder Wochen nach dem Färbevorgang.

Der Begriff Färbevorgang umfaßt dabei alle dem Fachmann bekannten Verfahren, bei denen auf das, gegebenenfalls angefeuchtete. Haar ein Färbemittel aufgebracht wird und dieses entweder für eine Zeit zwischen wenigen Minuten und ca. 45 Minuten auf dem Haar belassen und anschließend mit Wasser oder einem tensidhaltigen Mittel ausgespült wird oder ganz auf dem Haar belassen wird. Es wird in diesem Zusammenhang ausdrücklich auf die bekannten Monographien, z. B. das oben genannte Buch von Kh. Schrader, verwiesen, die das entsprechende Wissen des Fachmannes wiedergeben.

Die Zusammensetzung des Färbe- oder Tönungsmittels unterliegt keinen prinzipiellen Einschränkungen.
Als Farbstoff(vorprodukt)e werden
- Oxidationsfarbstoffvorprodukte vom Entwickler- und Kuppler-Typ und/oder
- natürliche und synthetische direktziehende Farbstoffe und/oder
- Vorstufen naturanaloger Farbstoffe ausgewählt aus der Gruppe 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie das 6-Hydroxyindolin, das 6-Aminoindolin und das 4-Aminoindolin eingesetzt werden..

Als Oxidationsfarbstoffvorprodukte vom Entwickler-Typ werden üblicherweise primäre aromatische Amine mit einer weiteren, in para- oder ortho-Position befindlichen, freien oder substituierten Hydroxy- oder Aminogruppe, Diaminopyndinderivate, heterocyclische Hydrazone, 4-Aminopyrazolderivate sowie 2,4,5,6-Tetraaminopyrimidin und dessen Derivate eingesetzt. Geeignete Entwicklerkomponenten sind beispielsweise p-Phenylendiamin, p-Toluylendiamin, p-Aminophenol, o-Aminophenol, 1-(2'-Hydroxyethyl)-2,5-diaminobenzol, N.N-Bis-(2-hydroxy-ethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxymethylamino-4-amino-phenol, Bis-(4-aminophenyl)amin, 4-Amino-3-fluorphenol, 2-Aminomethyl-4-aminophenol, 2-Hydroxymethyl-4-aminophenol, 4-Amino-2-((diethylamino)-methyl)-phenol, Bis-(2-hydroxy-5-aminophenyl)-methan, 1,4-Bis-(4-aminophenyl)-diazacycloheptan. 1,3-Bis(N(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 741 bzw. WO 94/08970 wie z. B. 4,5-Diamino-1-(2'-hydroxyethyl)-pyrazol. Besonders vorteilhafte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2-Aminomethyl-4-aminophenol, 2,4,5,6-Tetraaminopyrimidin, 2-Hydroxy-4,5,6-triaminopyrimidin, 4-Hydroxy-2,5,6-triaminopyrimidin.

Als Oxidationsfarbstoffvorprodukte vom Kuppler-Typ werden in der Regel m-Phenylendiaminderivate, Naphthole, Resorcin und Resorcinderivate, Pyrazolone und m-Aminophenolderivate verwendet. Beispiele für solche Kupplerkomponenten sind
- m-Aminophenol und dessen Derivate wie beispielsweise 5-Amino-2-methylphenol. 5-(3-Hydroxypropylamino)-2-methylphenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-(Ethylamino)-4-methylphenol und 2,4-Dichlor-3-aminophenol.
- o-Aminophenol und dessen Derivate,
- m-Diaminobenzol und dessen Derivate wie beispielsweise 2,4-Diaminophenoxyethanol, 1,3-Bis-(2,4-diaminophenoxy)-propan, 1-Methoxy-2-amino-4-(2'-hydroxyethylamino)benzol, 1,3-Bis-(2,4-diaminophenyl)-propan, 2,6-Bis-(2-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin. 2.6-Diarninopyridin, 2,3-Diamino-6-methoxypyridin und 3,5-Diamino-2.6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin.
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol,
Besonders geeignete Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2.5-Dimethylresorcin und 2,6-Dihydroxy-3,4-dimethylpyridin.

Direktziehende Farbstoffe sind üblicherweise Nitrophenylendiamine, Nitroaminophenole. Azofarbstoffe, Anthrachinone oder Indophenole. Besonders geeignete direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(β-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol.

In der Natur vorkommende direktziehende Farbstoffe sind beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267: Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsuntemehmen für Arzneimittel, Reformwaren und Körperpflegemittel e.V.. Mannheim, Bezug genommen.

Sowohl die Oxidationsfarbstoffvorprodukte als auch die direktziehenden Farbstoffe sind in den erfindungsgemäßen Mitteln bevorzugt in Mengen von 0,01 bis 20 Gew.-%, vorzugsweise 0,5 bis 5 Gew.-%, jeweils bezogen auf das gesamte Mittel, enthalten.

Als Vorstufen naturanaloger Farbstoffe werden Indoline wie sie in Anspruch 1 gennant werden eingesetzt.

Zu verwendende Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5.6-dihydroxyindolin und insbesondere das 5.6-Dihydroxyindolin.

Die Indolin- Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indolin-Derivate sind in diesen üblicherweise in Mengen von 0,05-10 Gew.-%, vorzugsweise 0,2-5 Gew.-% enthalten.

Bei der Verwendung von Farbstoff-Vorstufen vom Indolin- Typ kann es bevorzugt sein, diese zusammen mit mindestens einer Aminosäure und/oder mindestens einem Oligopeptid einzusetzen. Bevorzugte Aminosäuren sind Aminocarbonsäuren, insbesondere α-Aminocarbonsäuren und ω-Aminocarbonsäuren. Unter den α-Aminocarbonsäuren sind wiederum Arginin, Lysin, Ornithin und Histidin besonders bevorzugt. Eine ganz besonders bevorzugte Aminosäure ist Arginin, insbesondere in freier Form, aber auch als Hydrochlorid eingesetzt.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch. d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl- , propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich.

Erfolgt die Ausbildung der eigentlichen Haarfarben im Rahmen eines oxidativen Prozesses, so können übliche Oxidationsmittel wie insbesondere Wasserstoffperoxid oder dessen Anlagerungsprodukte an Harnstoff. Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur Erzeugung von oxidierenden Per-Verbindungen eingesetzt werden als auch zur Verstärkung der Wirkung einer geringen Menge vorhandener Oxidationsmittel.
So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z. B. 1 % und weniger, bezogen auf das gesamte Mittel) Wasserstoffperoxid durch Peroxidasen zu verstärken.

Zweckmäßigerweise wird die Zubereitung des Oxidationsmittels dann unmittelbar vor dem Färben der Haare mit der Zubereitung mit den Farbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C, bevorzugt bei der Temperatur der Kopfhaut, liegen. Nach einer Einwirkungszeit von ca. 5 bis 45, insbesondere 15 bis 30. Minuten wird das Haarfarbemittel durch Ausspülen von dem zu färbenden Haar entfernt. Das Nachwaschen mit einem Shampoo entfällt, wenn em stark tensidhaltiger Träger, z. B. ein Färbeshampoo, verwendet wurde.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, daß dem Mittel bestimmte Metallionen zugesetzt werden. Solche Metallionen sind beispielsweise Zn²⁺, Cu²⁺ Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflußt werden.

Ein weiterer Gegenstand der Erfindung ist die Verwendung einer Zubereitung eines Oxidationsmittel zur Verbesserung der Waschechtheit von Färbungen keratinischer Fasern.

In einer bevorzugten Ausführungsform wird eine wäßrige Zubereitung eines Oxidationsmittel zur Verbesserung der Waschechtheit von Färbungen keratinischer Fasern verwendet.

Die folgenden Beispiele sollen die Erfindung näher erläutern.

### Beispiele

Alle Mengenangaben in den folgenden Beispielen sind, sofern nicht anders vermerkt, Gewichtsteile.

### 1. Rezepturen

### 1.1.1. Farbfixiermittel 1

| | |
|---|---|
| Carbopol®ETD 2050¹ | 0,7 |
| Dehydol®LS 4² | 1,5 |
| NaOH. 10%ig in Wasser | 0,7 |
| Ethylendiaminotetraessigsäure, Dinatriumsalz | 0,1 |
| Turpinal®SL³ | 1,0 |
| Wasserstoffperoxid (50%ig in Wasser) | 6,0 |
| Wasser | ad 100 |

| | |
|---|---|
| ¹ Acrylpolymer (INCI-Bezeichnung: Carbomer) (GOODRICH) | |
| ² C₁₂₋₁₄-Fettalkohol + 4 EO (INCI-Bezeichnung: Laureth-4) (HENKEL) | |
| ³ 1-Hydroxyethan-1,1-diphosphonsäure (ca. 60 % Aktivsubstanz in Wasser ; INCI-Bezeichnung: Etidronic Acid, Aqua) (HENKEL) Das Mittel hatte einen pH-Wert von 4,5 | |

### 1.1.2. Farbfixiermittel 2

| | |
|---|---|
| Carbopol®ETD 2050 | 0,7 |
| Dehydol®LS 4 | 1,5 |
| NaOH, 10%ig in Wasser | 0,7 |
| Ethylendiaminotetraessigsäure, Dinatriumsalz | 0,1 |
| Turpinal®SL | 1,0 |
| Wasserstoffperoxid (50%ig in Wasser) | 2,0 |
| Wasser | ad 100 |
| Das Mittel hatte einen pH-Wert von 4,5. | |

### 1.1.3. Farbfixiermittel 3

| | |
|---|---|
| Dehyquart®A⁴ | 2,0 |
| Stenol®1618⁵ | 1,5 |
| Paraffinöl perliquidum | 1,5 |
| Wasserstoffperoxid (50%ig in Wasser) | 2,0 |
| Ethylendiaminotetraessigsäure, Dinatriumsalz | 0,1 |
| Wasser | ad 100 |
| Das Mittel hatte einen pH-Wert von 5,0. | |

| | |
|---|---|
| ⁴ Trimethylhexadecylammoniumchlorid (ca. 25 % Aktivsubstanz in Wasser; INCI-Bezeichnung: Aqua, Cetrimoniumchloride) (HENKEL) | |
| ⁵ C₁₆₋₁₈-Fettalkohol (INCI-Bezeichnung: Cetearyl Alcohol) (HENKEL) | |

### 1.2.1. Färbecreme A

| | |
|---|---|
| Hydrenol®D⁶ | 9,0 |
| Lorol®techn.⁷ | 2,0 |
| Texapon®NSO⁸ | 26,0 |
| 5-Amino-6-chlor-2-methyl-phenol | 0,62 |
| 3-Methyl-4-aminophenol | 0,48 |
| 2-Amino-3-hydroxypyridin | 0,20 |
| Resorcin | 0,10 |
| p-Toluylendiaminsulfat | 0,72 |
| 1,2-Propylenglykol | 3,0 |
| Natriumsulfit | 0,5 |
| Ammoniak, 25 %ig in Wasser | 4,0 |
| Wasser | ad 100 |

| | |
|---|---|
| ⁶ Talgfettalkohol (INCI-Bezeichnung: Cetearyl Alcohol) (HENKEL) | |
| ⁷ C₁₂₋₁₈-Fettalkohol (INCI-Bezeichnung Coconut Alcohol) (HENKEL) | |
| ⁸ Laurylethersulfat-Natriumsalz (ca. 28 % Aktivsubstanz in Wasser; INCI-Bezeichnung: Sodium Laureth Sulfate) (HENKEL) | |

### 1.2.2. Färbecreme B

| | |
|---|---|
| Hydrenol®D | 9,0 |
| Lorol® techn. | 2,0 |
| Texapon®NSO | 26,0 |
| 4-Amino-2-hydroxytoluol | 0,15 |
| 2-Aminomethyl-4-aminophenol-hydrochlorid | 0,88 |
| Resorcin | 0,30 |
| p-Toluylendiaminsulfat | 0,61 |
| m-Aminophenol | 0,21 |
| 1,2-Propylenglykol | 3,0 |
| Ascorbinsäure | 0.5 |
| Ammoniak, 25 %ig in Wasser | 4,0 |
| Wasser | ad 100 |

### 1.2.3. Färbecreme C

| | |
|---|---|
| Hydrenol®D | 9,0 |
| Lorol® techn. | 2,0 |
| Texapön®NSO | 26,0 |
| 4-Amino-2-hydroxytoluol | 0,27 |
| Resorcin | 0.20 |
| p-Toluylendiaminsulfat | 0,44 |
| 4-Hydroxypropylamino-3-nitro-phenol | 0,27 |
| Ascorbinsäure | 0,4 |
| Natriumsulfit | 0,2 |
| Ammoniak, 25 %ig in Wasser | 4,5 |
| Wasser | ad 100 |

### 2. Vorbehandlung der Haarsträhnen

Die Ausfärbungen erfolgten auf Haarsträhnen der Fa. Kerling, 80% meliert. Die Strähnen hatten eine Länge von ca. 18 cm und ein Gewicht von ca. 2g.

Die abgebundene untere Hälfte wurde mit 7 g des mit 1,5 Gew.-% Natrosol® 250 HR (Hydroxyethylcellulose, Fa. Hercules) angedickten Handelsproduktes Poly Lock bei 32 °C in einem Umlufttrockenschrank 30 Minuten behandelt. Anschließend wurde die Strähnenhälfte mit warmem Wasser gespült und dann 10 min. mit 7g des Handelsproduktes Poly Lock Fixieremulsion behandelt, ausgewaschen und getrocknet. Dann wurde die behandelte Strähnenhälfte mit 4 g des Handelsproduktes Poly Blond Ultra 30 Minuten bei 32 °C ultrablondiert. Schließlich wurde die Strähnenhälfte erneut gewaschen, getrocknet und nochmals wie oben beschrieben, einer Kaltwelle unterzogen. Schließlich wurde die gesamte Haarsträhne mit 9 g des Handelsproduktes Poly Blond Ultra ultrablondiert. (30 Minuten bei 32°C).

### 3. Coloration und Nachbehandlung

Die jeweilige Färbecreme wurde 1: 1 mit einer 6%igen wäßngen H₂O₂ - Emulsion wie sie in Handelsprodukten der Poly Color Brillance - Serie enthalten ist, gemischt und auf die nach dem oben beschriebenen Verfahren vorbehandelten Strähnen aufgetragen. Die Einwirkzeit betrug 30 Minuten bei 32 °C. Danach wurde die Färbecreme mit Wasser ausgespült.

Im Anschluß daran wurde das Farbfixiermittel auf die feuchte Haarsträhne aufgetragen und nach einer Einwirkzeit von 5 Minuten bei 32°C mit Wasser wieder abgespült. Die Strähne wurde danach getrocknet, mit dem Farbmetrikgerät textflash der Fa. Datacolor vermessen und anschließend 6 mal mit Poly Kur Aloe & Mango Balsam Shampoo shampooniert.

Nach dem Trocknen wurde die Strähne erneut farbmetrisch vermessen und mit dem Color Tools Quality Programm Version 1.3 die Farbstärke im oberen und unteren Bereich im Vergleich zur ungewaschenen Haarsträhne bestimmt.

Zum Vergleich wurde mit jeder Farbmischung auch eine Haarsträhne ausgefärbt, die nicht mit dem Farbfixierungsmittel nachbehandelt wurde. Auch diese Strähne wurde vor und nach dem sechsmaligen Shampoonieren farbmetrisch vermessen und die Farbstärke im oberen und unteren Teil der Strähne bestimmt.

### 4. Ergebnisse

| Färbecreme | Farbfixiermittel | Farbstärke auf dem | |
|---|---|---|---|
| | | oberen | unteren |
| | | Teil der Strähne | |
| A | 1 | 98 % | 86 % |
| A | 2 | 98 % | 81 % |
| A | - | 93 % | 64 % |
| B | 1 | 95 % | 89 % |
| B | 2 | 95 % | 93 % |
| B | - | 87 % | 52 % |
| C | 3 | 81 % | 58 % |
| C | - | 81 % | 33 % |

Wie aus den Ergebnissen ersichtlich ist, wiesen die Färbungen, bei denen die Strähnen nicht nach dem erfindungsgemäßen Verfahren zur Verbesserung der Waschechtheit gefärbt wurden, eine deutlich schlechtere Waschechtheit auf.

## Patentansprüche

1. Verfahren zur Färbung keratinischer Fasern, **dadurch gekennzeichnet, daß** in einem ersten Schritt die keratinischen Fasern in üblicher Weise mit einem Färbemittel enthaltend
• Oxidationsfarbstoffvorprodukte vom Entwickler- und Kupplertyp und/oder
• natürliche und/oder synthetische direktziehende Farbstoffe und/oder
• Vorstufen naturanaloger Farbstoffe ausgewählt aus der Gruppe 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin sowie das 6-Hydroxyindolin, das 6-Aminoindolin und das 4-Aminoindolin,
gefärbt werden und in einem zweiten Schritt eine Zubereitung als Farbfixiermittel auf die keratinischen Fasern aufgetragen wird, das als Oxidationsmittel 0,1 - 10 Gew.-%, bezogen auf das gesamte Mittel, Wasserstoffperoxid oder eine organische Verbindung enthält, wobei das Farbfixiermittel unmittelbar nach der Anwendung oder einer kurzen Einwirkzeit wieder ausgespült wird.

2. Verfahren nach Anspruch 1, **dadurch gekennzeichnet, daß** das Oxidationsmittel Wasserstoffperoxid ist.

3. Verfahren nach Anspruch 1, **dadurch gekennzeichnet, daß** die organische Verbindung ausgewählt ist aus der Gruppe, die von Benzochinonen, Percarbamid und Melaminperhydrat gebildet wird.

4. Verfahren nach einem der Ansprüche 1 bis 3, **dadurch gekennzeichnet, daß** es sich um eine wäßrige Zubereitung handelt.

5. Verfahren nach einem der Ansprüche 1 bis 4, **dadurch gekennzeichnet, daß** die Zubereitung weiterhin ein Tensid enthält.

6. Verfahren nach einem der Ansprüche 1 bis 5, **dadurch gekennzeichnet, daß** die Zubereitung weiterhin eine Wachs- oder Ölkomponente enthält.

7. Verfahren nach einem der Ansprüche 1 bis 6, **dadurch gekennzeichnet, daß** die Zubereitung weiterhin einen Pflegestoff enthält.

8. Verfahren nach einem der Ansprüche 1 bis 7, **dadurch gekennzeichnet, daß** die Einwirkzeit 1 bis 15 Minuten beträgt.

9. Verwendung einer Zubereitung eines Oxidationsmittels zur Verbesserung der Waschechtheit von Färbungen keratinischer Fasern.

10. Verwendung nach Anspruch 9, **dadurch gekennzeichnet, daß** es sich um eine wäßrige Zubereitung handelt.

## Claims

1. A process for colouring keratinous fibres, **characterized in that**, in a first step, the keratinous fibres are coloured in the usual way with a colorant containing
• oxidation dye precursors of the primary intermediate and secondary intermediate type and/or
• natural and/or synthetic substantive dyes and/or
• precursors of nature-analogous dyes selected from the group consisting of 5,6-dihydroxyindoline, N-methyl-5,6-dihydroxyindoline, N-ethyl-5,6-dihydroxyindoline, N-propyl-5,6-dihydroxyindoline, N-butyl-5,6-dihydroxyindoline and 6-hydroxyindoline, 6-aminoindoline and 4-aminoindoline,
and, in a second step, a preparation containing 0.1 to 10% by weight - based on the colorant as a whole - of hydrogen peroxide or an organic compound is applied to the keratinous fibres as a colour fixing medium, the colour fixing medium being rinsed out again immediately after use or after a short contact time.

2. A process as claimed in claim 1, **characterized in that** the oxidizing agent is hydrogen peroxide.

3. A process as claimed in claim 1, **characterized in that** the organic compound is selected from the group consisting of benzoquinones, percarbamide and melamine perhydrate.

4. A process as claimed in any of claims 1 to 3, **characterized in that** the preparation is a water-containing preparation.

5. A process as claimed in any of claims 1 to 4, **characterized in that** the preparation additionally contains a surfactant.

6. A process as claimed in any of claims 1 to 5, **characterized in that** the preparation additionally contains a wax or oil component.

7. A process as claimed in any of claims 1 to 6, **characterized in that** the preparation additionally contains a care component.

8. A process as claimed in any of claims 1 to 7, **characterized in that** the contact time is 1 to 15 minutes.

9. The use of a preparation of an oxidizing agent for improving the fastness to washing of colours on keratinous fibres.

10. The use claimed in claim 9, **characterized in that** the preparation is a water-containing preparation.

## Revendications

1. Procédé pour la teinture de fibres kératiniques, **caractérisé en ce que** les fibres kératiniques sont teintes dans une première étape de manière usuelle avec un agent de teinture contenant
• des précurseurs de colorants d'oxydation du type agent de développement et de couplage et/ou
• des colorants naturels et/ou synthétiques montant directement sur les fibres et/ou
• des précurseurs de colorants analogues aux colorants naturels, choisis dans le groupe constitué par la 5,6-dihydroxyindoline, la N-méthyl-5,6-dihydroxyindoline, la N-éthyl-5,6-dihydroxyindoline, la N-propyl-5,6-dihydroxyindoline, la N-butyl-5,6-dihydroxyindoline ainsi que la 6-hydroxyindoline, la 6-aminoindoline et la 4-aminoindoline,
et dans une deuxième étape, une composition est appliquée sur les fibres kératiniques en tant que fixateur de la teinte, qui contient comme oxydant 0,1 à 10% en poids par rapport à l'agent total de peroxyde d'hydrogène ou d'un composé organique, le fixateur de la teinte étant éliminé par rinçage immédiatement après l'application ou après un court laps de temps d'action.

2. Procédé selon la revendication 1, **caractérisé en ce que** l'oxydant est le peroxyde d'hydrogène.

3. Procédé selon la revendication 1, **caractérisé en ce que** le composé organique est choisi dans le groupe formé par les benzoquinones, le percarbamide et la mélamine perhydratée.

4. Procédé selon l'une quelconque des revendications 1 à 3, **caractérisé en ce qu'**il s'agit d'une composition aqueuse.

5. Procédé selon l'une quelconque des revendications 1 à 4, **caractérisé en ce que** la composition contient en outre un agent tensioactif.

6. Procédé selon l'une quelconque des revendications 1 à 5, **caractérisé en ce que** la composition contient en outre un composant cireux ou huileux.

7. Procédé selon l'une quelconque des revendications 1 à 6, **caractérisé en ce que** la composition contient en outre une substance de soin.

8. Procédé selon l'une quelconque des revendications 1 à 7, **caractérisé en ce que** le laps de temps d'action est de 1 à 15 minutes.

9. Utilisation d'une composition d'un oxydant pour améliorer la résistance au lavage de teintures de fibres kératiniques.

10. Utilisation selon la revendication 9, **caractérisée en ce qu'**il s'agit d'une composition aqueuse.
